# EUROPEAN PATENT APPLICATION

(11) **EP 1 108 430 A1**
(43) Date of publication of application: **20.06.2001**
(21) Application number: 99938593.3
(22) Date of filing: 24.08.1999
(51) Int. Cl.: A61K 31/505

(54) **CALCIUM CHANNEL ANTAGONISTS**

(30) Priority: 25.08.1998 JP 23844898
(71) Applicant: Nippon Shinyaku Co., Ltd., Kyoto-shi Kyoto 601-8550 (JP)
(72) Inventor: ITO, Yoshinori, Moriyama-shi, Shiga 524-0041 (JP)
(74) Representative: VOSSIUS & PARTNER
(86) International application number: JP9904535
(87) International publication number: WO0010571

(57) **Abstract**

P/Q-type calcium channel blockers, inhibitors against neurotransmitter release via P/Q-type calcium channel and chronic pain lenitives, containing as the active ingredient a compound represented by general formula (I) or salts thereof.

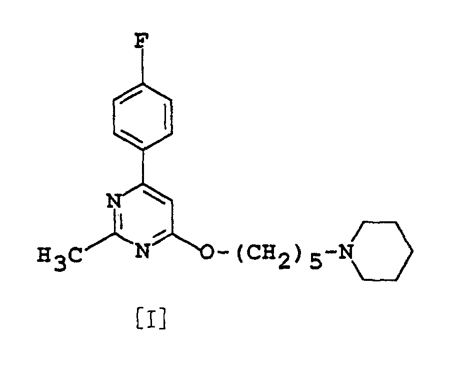

## Description

### TECHNICAL FIELD

The present invention relates to a P/Q-type calcium channel blocker.

### BACKGROUND ART

The voltage-dependent calcium channel in the plasma membrane opens on depolarization of the membrane potential to selectively admit an influx of extracellular calcium ions which depends on the electrochemical gradient. Thus, the calcium channel has a dual role to play, namely the role of generating an action potential and the role of converting the membrane potential information to chemical information which is a change in calcium ion concentration. Electrophysiologically the calcium channels are classified into two major groups according to the permeation pore-opening potential, namely the high voltage-activated channel (which does not open until the membrane potential has been depolarized close to 0 mV) and the low voltage-activated channel (which opens even when the membrane potential is fairly negative at about -60 mV). As the calcium channel, 6 subtypes (L, N, P, Q, T and R) have been identified to this day. The high voltage-activated channel has so far been classified into L, N and P subtypes according to electrophysiological and pharmacological properties. The L-type channel is characterized as a dihydropyridine (DHP)-sensitive high voltage-activated Ca²⁺ channel and exists in the smooth muscles of peripheral tissues such as blood vessels, urinary bladder and digestive organs as well as in the nerves and myocardium. The N-type channel is characterized as a non-DHP-sensitive high voltage-activated Ca²⁺ channel and exists at nerve terminals but does not exist in smooth muscles. The P-type channel is expressed in cerebellar Purkinje cells at a high level and, together with the Q-type channel which is said to be its subspecies, exists at the nerve terminal to regulate the release of neurotransmitters. Both P-type and Q-type channels are insensitive to ω-conotoxin GVIA and are blocked by ω-agatoxin IVA and low-molecular-weight polyamine FTX (funnel-web spider toxin) which are components of spider toxin. So far, between the two types of channels, there has been recognized only a quantitative difference in the sensitivity to ω-agatoxin IVA and it remains to be clarified whether the Q-type is identical to the P-type. Generally, therefore, both channels are collectively called the "P/Q-type calcium channel". The calcium channel called R-type exists in granule cells of the cerebellum. The N, P, Q and R-type channels can be lumped together as the non-L-type channel, and all of them are distributed in abundance at nerve terminals, reportedly contributing to the regulation of neurotransmitter release. As the low voltage-activated channel, only the T-type is known. The T-type channel is broadly distributed in various tissues and involved in pacemaker activity and excitability in the heart muscle and nerves.

As mentioned above, the P and Q-type Ca²⁺ channels are richly distributed at nerve terminals, and activation of these channels triggers an influx of Ca²⁺ ions into the nerve terminals and, hence, a release of neurotransmitters. Therefore, any compound causing a P- and/or Q-type Ca²⁺ channel blockade suppresses the release of neurotransmitters and, thus, can be used as a therapeutic agent for various neurotransmitter-associated diseases.

It is considered that in neuropathic pain and rheumatism, for instance, the N-type and P/Q-type Ca²⁺ channels of the spinal cord sensory nerve are activated to cause an increased release of substance P and glutamic acid which are known to be transmitters of pain so that hyperalgesia is induced. It is known that P/Q -type Ca²⁺ channel blockers are useful in controlling chronic pain in those diseases.

Furthermore, the activation of calcium channels due to abnormal excitation of nerves and the consequent excessive release of neurotransmitters have been considered to be a factor in various types of neuropathy (epilepsy, psychosis, ALS) and cardiovascular diseases (hypertension, arrhythmia, myocardial infarction). Therefore, such blockers are expected to find application as therapeutic drugs for such diseases.

As a reagent having P- and/or Q-type Ca²⁺ channel blocker activity, ω-agatoxin IVA is commercially available. It is known that ω-agatoxin IVA, which is a peptide, alters the pain threshold to relieve chronic pain. The transfer of peptides to the central nervous system is generally poor and when administered orally, these substances are rapidly decomposed by peptidases. Therefore, in order that their action may be expressed and kept long, those peptides must be administered sustainedly in large doses.

The anti-inflammatory/analgesics in routine use are capable of controlling inflammatory responses to relieve pain and swelling but may hardly arrest chronic pain. Moreover, since prolonged administration entails side effects, none of them is a fully satisfactory drug. For this reason. a real need has been felt for a chronic pain-relieving drug having a novel mode of action differentiating it from the anti-inflammatory/analgesics mentioned above.

The compound of the following formula [I] according to the present invention, namely 4-(4-fluorophenyl)-2-methyl-6-(5-piperidinopentyloxy)pyrimidine [hereinafter abbreviated as Compound 1], 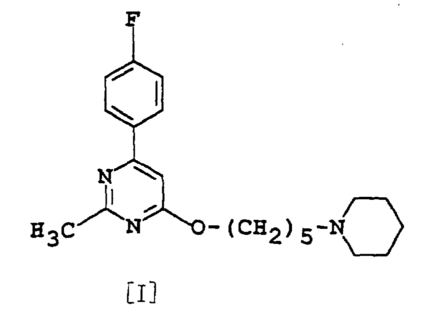 is known to inhibit neuronal death following subarachnoid hemorrhage or brain ischemia (WO 96/07641). It is also reported that the compound has sodium ion channel blocker activity and N-, L- and T-type calcium channel blocker activities as well (Japanese Journal of Pharmacology 73 (Supplement I), page 193, 1997).

### DISCLOSURE OF INVENTION

In the above perspective, the present inventors explored into a broad spectrum of compounds in search for a novel calcium antagonist acting on P/Q-type Ca²⁺ channels and discovered P/Q-type Ca²⁺ channel blocker activity in the above Compound 1 and its salt. The present invention has accordingly been developed.

The present invention, thus, is concerned with the following (1)∼(4).
(1) A P/Q-type Ca²⁺ channel blocker comprising Compound 1 or a salt thereof as an active ingredient.
(2) An inhibitor of P/Q-type Ca²⁺ channel-mediated release of neurotransmitters which comprises Compound 1 or a salt thereof as an active ingredient.
(3) An inhibitor of P/Q-type Ca²⁺ channel-mediated dopamine release which comprises Compound 1 or a salt thereof as an active ingredient.
(4) A chronic pain-relieving agent which comprises Compound 1 or a salt thereof as an active ingredient.

The present invention is characterized by its being grounded on the finding that Compound 1 and its salt have P/Q-type Ca²⁺ channel blocker activity, which is distinct from their hitherto-known brain neuronal death-inhibiting activity, Na⁺ channel blocker activity and N-, L-, and T-type Ca²⁺ channel blocker activities.

The present invention is now described in detail.

The term "P/Q-type calcium ion (Ca²⁺) channel blocker" means a drug that blocks P-type and/or Q-type Ca²⁺ channels at nerve terminals to inhibit release of neurotransmitters and, as such, can be used in the therapy of various neurotransmitter-associated diseases.

The term "an inhibitor of neurotransmitter release" means a drug which inhibits the release of various transmitters from nerve terminals in the central nervous system or the peripheral nervous system inclusive of sensory and autonomic nerves.

The term "chronic pain-relieving agent" means a drug which improves the pain threshold depressed by irritation of sensory nerves in an inflammatory phase of neuropathic pain or rheumatism to relieve chronic pain.

The salt of Compound 1 includes the salts of inorganic acids such as hydrochloric acid, sulfuric acid, nitric acid, phosphoric acid, hydrofluoric acid and hydrobromic acid, and the salts of organic acids such as acetic acid, tartaric acid, lactic acid, citric acid, fumaric acid, maleic acid, succinic acid, methanesulfonic acid, ethanesulfonic acid, benzenesulfonic acid, toluenesulfonic acid, naphthalenesulfonic acid and camphorsulfonic acid, among others.

Compound 1 and its salt can be produced by, for example, the process disclosed in WO 96/07641.

For use as a P/Q-type Ca²⁺ channel blocker, an inhibitor of neurotransmitter release, or a chronic pain-relieving agent, Compound 1 or a salt thereof can be formulated as a pharmaceutical composition containing it in a proportion of, for example, 0.1 - 99.5 weight %, preferably 0.5 - 90 weight %, in a pharmaceutically acceptable nontoxic, inert carrier and administered to mammals inclusive of humans.

Examples of carrier usable include solid, semisolid, or liquid diluents, fillers, and other formulation auxiliaries, and at least one of them is employed. The pharmaceutical composition is preferably administered in a unit dosage form. The pharmaceutical composition of the present invention can be administered orally or parenterally (*e*.*g*. injection or rectally). A dosage form suited for each administration mode is of course employed. For instance, the oral administration is preferred.

The dosage of the compound as a P/Q-type calcium channel blocker, a neurotransmitter release-inhibitor or a chronic pain-relieving agent should preferably be adjusted in consideration of conditions of the patient such as age, body weight, nature and severity of disease, as well as the route of administration, but the daily dosage of the compound of the present invention as an active ingredient for adult for oral administration can generally be 0.1 mg - 500 mg, preferably 1 mg - 200 mg. The dose range above is not critical and a lower dosage may be sufficient in some cases, while a higher dosage beyond the said range may be needed in other cases. The daily dosage can be administered in divided dosage.

Furthermore, Compound 1 and its salt have not only sodium channel blocker activity but also N-, L- and T-type calcium channel blocker activities and, therefore, can be used in various cardiovascular diseases (e.g. arrhythmia, angina pectoris).

Oral administration can be carried out using a solid or liquid unit dosage form, such as bulk powders, powders, tablets, dragees, capsules, granules, suspensions, solutions, syrups, drops, sublingual tablets and other forms. Bulk powders are prepared by comminuting Compound 1 or its salt to a suitable particle size. Powders are prepared by comminuting Compound 1 or its salt to a suitable size and blending the resulting powder with similarly comminuted pharmaceutical carriers such as edible carbohydrates, *e.g.* starch, mannitol, *etc.,* and other substances, if any. Where necessary, flavorants, preservatives, dispersing agents, colorants, perfumes, *etc.* can be added.

Capsules are manufactured by encapsulating comminuted bulk particles, powders, or granules obtained in the manner described below for tablets in gelatin or other capsule shells. A lubricant or fluidizing agent, *e.g.* colloidal silica, talc, magnesium stearate, calcium stearate, solid polyethylene glycol, *etc.,* may be added to the powdery materials prior to encapsulation. The medicinal efficacy of a capsule after ingestion may be improved by adding a disintegrator or a solubilizer. *e.g.* carboxymethylcellulose, carboxymethylcellulose calcium, low-substitution-degree hydroxypropylcellulose, croscarmellose sodium, carboxymethylstarch sodium, calcium carbonate, and sodium carbonate.

The finely pulverized powder of Compound 1 or its salt may be suspended and dispersed in vegetable oil, polyethylene glycol, glycerin or a surfactant and packaged in gelatin sheet to provide soft capsules.

Tablets can be manufactured by preparing a powdery composition, granulating or slugging it, adding a disintegrator or a lubricant thereto, and compressing the mixture.

The powdery composition can be prepared by mixing a properly comminuted substance with a diluent or a base mentioned above, which may further contain, where necessary, a binder (*e.g*. carboxymethylcellulose sodium, methylcellulose, hydroxypropylmethylcellulose, gelatin, polyvinylpyrrolidone, polyvinyl alcohol, *etc*.), a dissolution retardant (*e.g*, paraffin), a reabsorptioner (*e.g*. quartenary salts), and an adsorbent (*e*.*g*. bentonite, kaolin, dicalcium phosphate, *etc.).* The powdery composition can be granulated by wetting the material with a binder, *e.g*. a syrup, a starch paste, a solution of gum arabic or cellulose, or a polymer solution, mixing with stirring, drying and then purvelizing. The powder, without being granulated, can be compressed with a tablet machine to give slugs of crude form, which is then crushed to give granules. The granules thus obtained can be protected against inter-adhesion by adding a lubricant such as stearic acid, a salt of stearic acid, talc or mineral oil. The lubricated granules are then compressed into tablets. The resulting bare tablets can be film-coated or sugar-coated.

Compound 1 or its salt may be directly compressed after mixing with a free-flowing inert carrier without being subjected to the above granulation or slugging process. A transparent or translucent protective coat comprising a hermetic shellac film, a sugar or polymer coat, or a wax glaze coat may also be applied. Other oral dosage forms such as solutions, syrups and elixirs can also be provided in unit dosage forms each containing a predetermined amount of the drug. A syrup can be manufactured by dissolving Compound 1 or its salt in a suitable pleasantly flavored aqueous vehicle, while an elixir can be manufactured using a nontoxic alcoholic vehicle. Suspensions are prepared by dispersing Compound 1 or its salt in nontoxic carriers. Solubilizers, emulsifiers (for example, ethoxylated isostearyl alcohols, and polyoxyethylene sorbitol esters), preservatives, flavors (for example, peppermint oil and saccharin), or the like may be added, if necessary.

Where necessary, a unit dose formulation for oral administration may be microencapsulated. This formulation can also be coated with, or embedded in, a polymer, a wax, or the like to provide a prolonged action or sustained release of active ingredient.

Injections and suppositories can be used for parenteral administration. Said administration can be carried out using a liquid unit dosage form, for subcutaneous, intramuscular or intravenous administration, such as a solution or a suspension. Such a dosage form can be prepared by dissolving or suspending a predetermined amount of Compound 1 or its salt in a nontoxic liquid vehicle for injection, such as an aqueous or oily vehicle, and sterilizing the solution or the suspension. To isotonize an injection, a nontoxic salt or salt solution can be added. In addition, stabilizers, preservatives, emulsifiers, and other additives may also be concomitantly used.

Rectal administration can be carried out by using suppositories which can be manufactured by mixing or suspending or its salt with a water-soluble or -insoluble low-melting solid base, such as polyethylene glycol, cacao butter, semi-synthetic fats and oils (for example, Witepsol (registered trade mark)), or a higher ester (*e.g.* myristyl palmitate), or a mixture of them.

The P/Q-type Ca²⁺ channel blocking action, P/Q-type Ca²⁺ channel-mediated transmitter release-inhibitory action, and chronic pain-relieving action of Compound 1 and its salt were confirmed in the experiments performed with reference to the following information.

As the nerve cell membrane is depolarized by a high-potassium stimulus, the calcium channel is activated to induce an intracellular influx of calcium ions. As the intracellular Ca²⁺ ion concentration is increased, nitric oxide synthase (NOS), which is a calcium-dependent enzyme, is activated in the nerve cell. Moreover, from the nerve terminal, neurotransmitters are released. Therefore, using cultured nerve cells, the intracellular calcium ion concentration and NOS activity under high-potassium stimulation were studied. Furthermore, as it has been confirmed that the N-type and P/Q-type calcium channels are involved in the release of dopamine from the nerve terminal under high K⁺ stimulation, the dopamine release was measured in vivo.

### BRIEF DESCRIPTION OF THE DRAWINGS

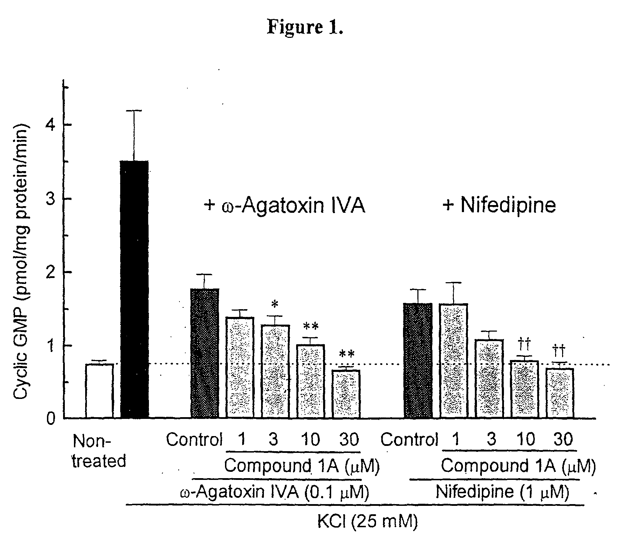

Fig. 1 shows the effect of Compound 1 hydrochloride (hereinafter referred to as Compound 1A) on the KCl-evoked NOS activation in cultured mouse cerebral cortex-derived neurons. Each value stands for mean ± S.E. (N = 5∼6). The marks ^{*} (5% level) and ^{**} (1% level) indicate significant difference between a ω-agatoxin IVA-treated group and a control group (Dunnett's test). The mark †† (1% level) indicates significant difference between a nifedipine-treated group and a control group (Dunnett's test).

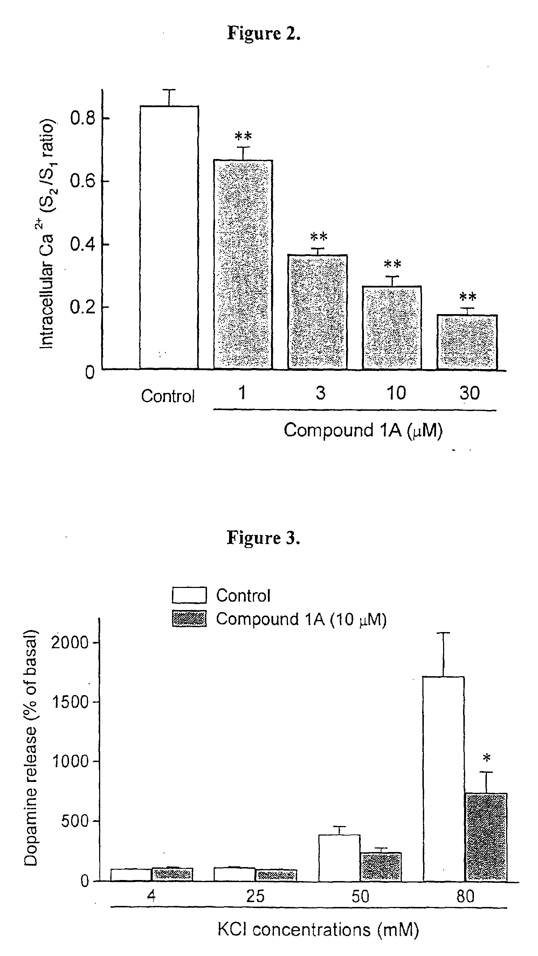

Fig. 2 shows the effect of Compound 1A on the free calcium ion concentration in cultured mouse cerebral cortex-derived neurons. The ordinate represents the ratio (S2/S1) of increase in Ca²⁺ concentration in response to serial stimulations. Each value stands for mean ± S.E. The mark ^{**} indicates significant difference from a control group (1% level, Dunnett's test).

Fig. 3 shows the effect of Compound 1A on the dopamine release from the rat striatum as elicited by high-potassium stimulation. The shaded bar represents the dopamine release in the presence of Compound 1A and the open bar represents the dopamine release in the absence of Compound 1A. Each value stands for mean ± S.E. The mark ^{*} indicates significant difference from the control group (5% level, t-test).

### BEST MODE FOR CARRYING OUT THE INVENTION

The following test examples and formulation example illustrate the invention in further detail. It is to be understood that the invention is by no means limited to the specific formulation presented later herein. As the test drug, Compound 1 hydrochloride (Compound 1A) was used.

### Test Example 1

### Inhibitory effect on the activation of nitric oxide synthase (NOS) in mouse cerebral cortex-derived primary cultured neurons

Using the calcium-dependent enzyme NOS activity in mouse cerebral cortex-derived primary cultured neurons as a marker, the function of the neuron calcium channels was investigated and the effect of Compound 1A on the function was evaluated.

### Primary culture of neurons:

The neocortex was isolated from fetal mice on embryonic day 15, cut to thin slices, and incubated in 0,1% trypsin Ca²⁺-free Puck's solution (pH 7.4) at 37°C for 5 minutes. Then, ice-cold 20% horse serum-supplemented Dulbecco's Modified Eagle's minimum essential medium (DMEM) was immediately added for terminating the trypsin reaction. To the above DMEM, 10 mM N-tris(hydroxymethyl)methyl-2-aminoethanesulfonic acid (TES), 10 mM N-2-hydroxyethylpiperazine-N'-2-ethanesulfonic acid (HEPES), 30 mM glucose, carbenicillin (0.1 mg/ml) and streptomycin (0.1 mg/ml) were added. The cells thus obtained were suspended in DMEM to prepare a cell suspension containing 1.45×10⁶ cells per ml. This suspension was seeded, 1.5 ml, in a poly-L-lysine (5 µg/ml H₂O)-coated Coating Culture Plastic Dish (Falcon, Franklin Lakes) or Coating Glass Bottom Dish (made by Meridian Instruments Far East). The dish was allowed to sit under gassing with 95% O₂-5% CO₂ mixture at 37°C and 100% humidity for 15 - 30 minutes. Then, the DMEM in the dish was aspirated off and after addition of 15% fetal calf serum-DMEM to the dish, the cells were cultured for 3 days. On day 4 of culture, the medium was replaced with DMEM containing 20 µM cytosine-β-D-arabinofuranoside (Ara-C) and 15% horse serum and the cells were further cultured for 24 hours. On day 5 of culture, the medium was replaced with 15% horse serum-DMEM. Thereafter, the culture was continued with the medium replaced with fresh 15% horse serum-DMEM every 4 days.

### Assay of NOS activity:

Using the method of Dawson et al. (1991) as modified (Tatsumi et al., 1998), NOS activity was assayed using the potency to produce cyclic GMP as an indicator. The NOS activity was expressed in terms of cyclic GMP production and analyzed by Dunnett's test (randomized block method).

### Results:

NOS, which is known to be activated on high-potassium stimulation of neurons, was inhibited about 56% by the L-type Ca²⁺ channel blocker nifedipine at 1 µM, about 53% by the P/Q-type Ca²⁺ channel blocker ω-agatoxin IVA at 0.1 µM, and completely by the two blockers used together. However, the N-type Ca²⁺ channel blocker ω-conotoxin GVIA 5 µM had substantially no effect on the NOS activation by KC1 stimulation. It was, therefore, clear that the L-type and P/Q-type calcium channels are related to the high-potassium activation of NOS.

Then, for the purpose of elucidating the action of Compound 1A on the L-type and P/Q-type calcium channels, its effect on the KCl-induced NOS activation in the presence of an L-type Ca²⁺ channel blocker or a P/Q-type Ca²⁺ channel blocker was investigated. Compound 1A (3∼30 µM) caused significant (P<0.01) and concentration-dependent inhibitions (Fig. 1, Dunnett's test) of the NOS activation in the presence of 0.1 µM ω-agatoxin IVA (the response mediated by the L-type Ca²⁺ channel) and the NOS activation in the presence of 1 µM nifedipine (the response mediated by the P/Q-type Ca²⁺ channel). Particularly at the concentration of 10 µM, the response mediated by the P/Q-type Ca²⁺ channel was completely inhibited.

Thus, the compound of the invention showed P/Q-type Ca²⁺ channel blocker activity in addition to L-type Ca²⁺ channel blocker activity.

### Test Example 2

### Inhibitory effect on the elevation of intracellular calcium ion concentration in mouse cerebral cortex-derived primary cultured neurons

To demonstrate that the inhibitory effect of Compound 1A on the high-potassium-evoked NOS activity is ascribable to inhibition of an intracellular influx of calcium ions through the calcium channel, the intracellular calcium ion concentration was measured by the 2-wavelength fluorescence method using fura-2 acetoxymethyl ester (fura-2/AM), a fluorescent probe for calcium, to study the effect on the elevation of intracellular free calcium ion concentration under high-potassium stimulation.

In the dish (the bottom opening fitted with cover glass) used for the measurement of calcium ion concentration, neurons on day 5 of culture were allowed to take up the fluorescent calcium probe fura-2/AM before determination. Thus, cultured neurons were incubated in 10 mM HEPES-NaOH buffer (pH 7.05) containing 10 µM fura-2/AM, 5.4 mM KC1, 130 mM NaCl, 2.5 mM CaCl₂ and 5.5 mM D-glucose at 37°C for 30 minutes to let fura-2/AM taken up in the cells. Thereafter, incubation was further continued for 30 minutes, whereby the fura-2/AM taken up in the cells was converted to fura-2 by endogenous esterase activity.

The number of neurons used in the experiment was 53 cells in a control group and, among Compound 1A groups, the number was 73 cells in the 1 µM group, 65 cells in the 3 µM group, 65 cells in the 10 µM group, and 74 cells in the 30 µm group.

The calcium ion concentration was determined by the method of Grynkiewicz et al. (1985). Thus, by fluorescent microscope-image processing, the calcium ion concentration was determined from the fluorescence intensities of fura - 2/Ca²⁺ at the exciting wavelengths of 340 nm and 380 nm. Using Argus-50/CA System (Hamamatsu Photonics), a calibration curve constructed beforehand was transformed to find the intracellular calcium ion concentration from the fluorescence intensity ratio.

The high-potassium stimulus was given twice, 15 minutes apart and for 15 seconds each, and the ratio of increase in free calcium ion concentration in response to the stimulations was calculated. Compound 1A was added 10 minutes before the second stimulation till the end of stimulation. As a result, Compound 1A (1 - 30 µM) was found to inhibit the high-potassium-stimulated increase in intracellular calcium ion concentration in a concentration-dependent manner (Fig. 2; Dunnett's test, p<0.01).

It became clear from the results of Test Examples 1 and 2 that, in primary cultured neurons, Compound 1A blocks the KCl·activated L-type and P/Q-type Ca²⁺ channels to suppress the influx of calcium ions into the cells and thereby inhibit the activation of NOS which is a calcium-dependent enzyme. Test Example 3

### Inhibitory effect on in vivo dopamine release from the rat striatum under high-potassium stimulation

Using dopamine, a representative neurotransmitter, the effect of Compound 1A on the inhibition of neurotransmitter release was studied.

The release of dopamine from the rat striatum was measured by the in vivo microdialysis method. A microdialysis probe fitted with a microinjection cannula was inserted into the striatum of an 8∼10·week·old male SD rat and the striatum was perfused with Ringer's solution at a rate of 2 µl/min.

Beginning 2 hours after the start of perfusion, the perfusate was recovered at a 20-minute interval and the dopamine in each perfusate was measured with a high-performance liquid chromatography-electrochemical detector. The drug was perfused into the striatum through the microdialysis probe or microinjected into the striatum via the microinjection cannula. The effect of the drug on the basal dopamine release was evaluated by comparing the values before and after stimulation and a statistical analysis was made by Dunnett's test. Then, to evaluate the effect of Compound 1A on KCl·stimulated dopamine release, the amount of dopamine released in the presence of Compound 1A (10 µM) (ipsilateral striatum) was compared with the amount of KCl-stimulated dopamine release in the absence of Compound 1A (contralateral striatum) and the difference was statistically analyzed by paired t-test.

While microinjection of ω-conotoxin GVIA (10 pmol/µl) and ω-agatoxin IVA (10 µM) caused a significant decrease (p<0.01) in dopamine release, this dopamine release was not influenced by perfusion with the L-type Ca²⁺ channel blocker nimodipine (1 pmol/µl). It, whereby, has been proved that the release of dopamine from the rat striatum in vivo is mediated by the N-type and P/Q-type Ca²⁺ channels.

Therefore, with a microdialysis probe inserted bilaterally, one of the striatum was perfused with Ringer's solution (control side) and the other striatum with Compound 1A (10 µM). K⁺ stimulation, 25∼80 mM, was consecutively given at a 20-minute interval. As a result, Compound 1A (10 µM) showed marked inhibitory effects on dopamine release with an increasing potassium concentration and specially inhibited dopamine release significantly at 80 mM KC1 (Fig. 3, p<0.05, paired t-test).

Thus, the compound of the invention blocked not only the L-type Ca²⁺ channel but also P/Q-type Ca²⁺ channel to suppress the release of neurotransmitters.

The results obtained in Test Examples 1∼3 indicate that the compound of the invention has P/Q-type Ca²⁺ channel blocker activity and P/Q-type Ca²⁺ channel-mediated neurotransmitter release-inhibitory activity.

### Formulation Example 1

### Tablets (oral tablets); 120 mg per tablet

| | |
|---|---|
| Compound 1A | 3 mg |
| Lactose | 58 mg |
| Corn starch | 30 mg |
| Crystalline cellulose | 20 mg |
| Hydroxypropylcellulose | 7 mg |
| Magnesium stearate | 2 mg |
| | 120 mg |

The above components are weighed in the indicated ratio and the components other than magnesium stearate are evenly kneaded together and prepared a granulation for compression using a machine for granulation. To the resulted granules is added magnesium stearate, and the mixture is compressed with a tablet machine to give tablets 8 mm in diameter and each weighing 120 mg for oral administration.

### INDUSTRIAL APPLICABILITY

Compound 1A has a potent P/Q-type Ca²⁺ channel blocking activity, is of low toxicity, and can be administered orally, so that it can be used as a therapeutic drug for chronic pain in neuropathic pain and rheumatism. Moreover, because the compound causes an N-type Ca²⁺ channel blockade as well, it can be used as a chronic pain-relieving agent in cancer- and AIDS-related pain, too. In addition, it finds application as a therapeutic drug for various types of neuropathy (epilepsy, psychosis, ALS, chronic pain in neuropathic pain and rheumatism, etc.) and cardiovascular diseases (hypertension, arrhythmia, myocardial infarction).

## Claims

1. A P/Q-type calcium channel blocker comprising a compound of the following formula [I] or a salt thereof as an active ingredient.

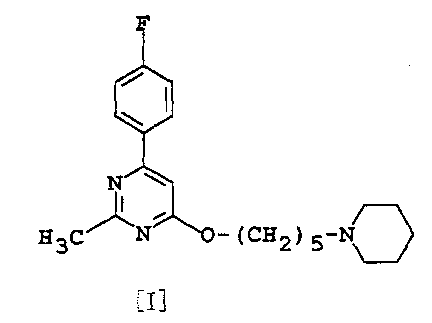

2. The P/Q-type calcium channel blocker as claimed in Claim 1 wherein the salt is the hydrochloride.

3. An inhibitor of P/Q-type calcium channel-mediated release of a neurotransmitter, which comprises the compound or salt mentioned in Claim 1 as an active ingredient.

4. An inhibitor of P/Q-type calcium channel-mediated release of dopamine, which comprises the compound or salt mentioned in Claim 1 as an active ingredient.

5. A chronic pain-relieving agent comprising the compound or salt of mentioned in Claim 1.
